(19) Europäisches Patentamt
European Patent Office
Office européen des brevets

(11) Veröffentlichungsnummer : **0 022 519**
**B1**

(12) # EUROPÄISCHE PATENTSCHRIFT

(45) Veröffentlichungstag der Patentschrift : **03.02.82**

(51) Int. Cl.³ : **C 07 C 39/11**, C 07 C 37/20

(21) Anmeldenummer : **80103762.3**

(22) Anmeldetag : **02.07.80**

(54) **Verfahren zur Herstellung eines Gemisches aus 2- und 4-Hydroxybenzylalkohol.**

(30) Priorität : **14.07.79 DE 2928554**

(43) Veröffentlichungstag der Anmeldung : **21.01.81 (Patentblatt 81/03)**

(45) Bekanntmachung des Hinweises auf die Patenterteilung : **03.02.82 Patentblatt 82/05**

(84) Benannte Vertragsstaaten : **BE CH DE FR GB IT LI NL**

(56) Entgegenhaltungen : **DE - A1 - 2 729 075**

(73) Patentinhaber : **BAYER AG**
**Zentralbereich Patente, Marken und Lizenzen**
**D-5090 Leverkusen 1, Bayerwerk (DE)**

(72) Erfinder : **Bauer, Kurt, Dr.**
**Corveyblick 41**
**D-3450 Holzminden (DE)**
Erfinder : **Mölleken, Reiner, Dr.**
**Bergstrasse 8**
**D-3450 Holzminden (DE)**
Erfinder : **Flege, Helmut, Dr.**
**Walter-Flex-Strasse 8**
**D-5090 Leverkusen (DE)**
Erfinder : **Wedemeyer, Karlfried, Dr.**
**Bilharzstrasse 7**
**D-5000 Köln (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 022 519 B1

Verfahren zur Herstellung eines Gemisches aus 2- und 4-Hydroxybenzylalkohol

Die Erfindung betrifft ein Verfahren zur Herstellung eines Gemisches aus 2- und 4-Hydroxybenzylalkohol.

Die Umsetzung von Phenol und Formaldehyd in Gegenwart stark basischer Katalysatoren ist bekannt. So ist z.B. in der UdSSR Zeitschrift Plast. Massy 1971 (6), S 11-13 (Referat siehe Chem. Abstr. Vol *75*, 77 564 t) ein Verfahren zur Herstellung von Phenol-Formaldehyd-Harzen durch Umsetzung von Phenol und Formaldehyd beschrieben, in dem als stark basische Katalysatoren Ammoniak bzw. Hexamethylentetramin eingesetzt werden. Wie aus der Untersuchung der Reaktionsprodukte hervorgeht, führt die Verwendung von Ammoniak bzw. Hexamethylentetramin zur Bildung von Aminverbindungen.

In der DE-OS 27 29 075 ist ein Verfahren zur Herstellung von 2- und 4-Hydroxybenzylalkohol-Gemischen beschrieben. Nach diesem Verfahren werden die Gemische in hohen Raum-Zeit-Ausbeuten durch Umsetzung von Phenol und Paraformaldehyd in Gegenwart stark basischer Katalysatoren erhalten.

In diesen Gemischen beträgt jedoch der Anteil des 4-Hydroxybenzylalkohols nur etwa 30 Gew.%, bezogen auf die im Reaktionsgemisch vorhandenen Hydroxybenzylalkohole.

Da 4-Hydroxybenzylalkohol ein wichtiges Ausgangsmaterial für die Herstellung von 4-Hydroxybenzaldehyd darstellt, bestand das Bedürfnis, Gemische von 2- und 4-Hydroxybenzylalkohol mit einem höheren Anteil an 4-Hydroxybenzylalkohol herzustellen.

Es wurde überraschenderweise gefunden, daß sich der Anteil an 4-Hydroxybenzylalkohol in besagten Hydroxybenzylalkohol-Gemischen wesentlich erhöhen läßt, wenn man die Umsetzung von Phenol mit Paraformaldehyd in Gegenwart basischer Verbindungen durchführt, die zwei oder mehr tertiäre Stickstoffatome im Molekül und einen $pK_a$-Wert $\geq 6{,}5$ (gemessen bei 20 °C in Wasser) aufweisen.

Die Erfindung betrifft daher ein Verfahren zur Herstellung von Gemischen aus 2- und 4-Hydroxybenzylalkohol durch Umsetzung von Phenol mit Paraformaldehyd in Gegenwart basischer Katalysatoren, das dadurch gekennzeichnet ist, daß man als basische Katalysatoren Verbindungen verwendet, die zwei oder mehr tertiäre Stickstoffatome im Molekül und einen $pK_a$-Wert $\geq 6{,}5$ (gemessen bei 20 °C in Wasser) aufweisen.

Der $pK_a$-Wert, der im Rahmen des erfindungsgemäßen Verfahrens zur Beschreibung der Basenstärke der zu verwendenden basischen Stickstoff-Verbindungen verwendet wird, ist der negative Logarithmus der sauren Dissoziationskonstanten $K_a$ der betreffenden Stickstoffverbindung ($pK_a = \log K_a$; Definition des $pK_a$ und der sauren Dissoziationskonstanten Ka siehe D. D. Perrin « Dissociation Constants of Organic Bases in Aqueous Solution » Butterworths London 1965 S. 1-4.). In diesem wie auch anderen Tabellenwerken sind zahlreiche für das erfindungsgemäße Verfahren geeignete Verbindungen und ihre $pK_a$-Werte angegeben. Verbindungen mit mehreren Stickstoffatomen besitzen bekanntlich mehrere $pK_a$-Werte. Maßgeblich im erfindungsgemäßen Sinne ist immer der höchste $pK_a$-Wert.

Als erfindungsgemäß zu verwendende Verbindungen mit mehreren tertiären Stickstoffatomen und einem $pK_a$-Wert $\geq$ (d.h. gleich oder größer als) 6,5 seien beispielsweise genannt:

a) peralkylierte offenkettige, monocyclische oder bicyclische Di- oder Polyamide und

b) N,N'-substituierte mono- und bicyclische Amidine.

Unter den Verbindungen des Typs A haben sich besonders solche peralkylierten offenkettigen, monocyclischen oder bicyclischen Di- oder Polyamine der Formeln

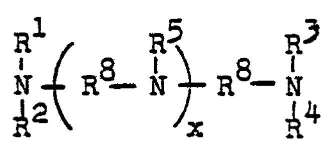

(I)

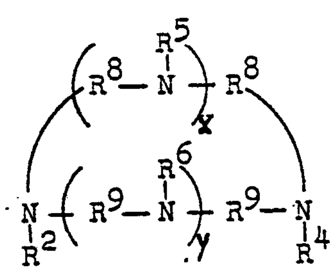

(II)

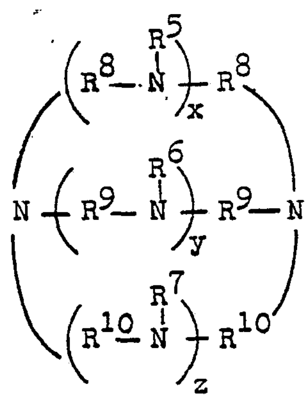

(III)

bewährt.

In den Formel I, II und III stehen

$R^1$, $R^2$, $R^3$, $R^4$, $R^5$, $R^6$, $R^7$ unabhängig voneinander für $C_1$-$C_{12}$-Alkyl, oder gegebenenfalls durch $C_1$-$C_4$-Alkylgruppen substituierte Cycloalkylgruppen

$R^8$, $R^9$ und $R^{10}$ unabhängig von einander für $C_2$-$C_6$-Alkylen- oder Cycloalkylengruppen und

x, y und z unabhängig von einander für eine ganze Zahl von 0 bis 10.

Unter den Verbindungen des Typs B haben sich besonders N,N'-substituierte mono- oder bicyclische Amidine der Formeln

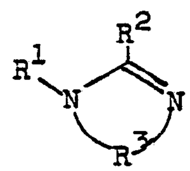

(IV)

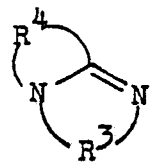

(V)

bewährt.

In den Formeln IV und V stehen

$R^1$ für eine $C_1$-$C_{12}$-Alkyl-, oder eine gegebenenfalls durch $C_1$-$C_4$-Alkylgruppen substituierte Cycloalkylgruppe

$R_2$ für Wasserstoff, eine $C_1$-$C_2$-Alkyl-, oder eine gegebenenfalls durch $C_1$-$C_4$-Alkylgruppen substituierte Cycloalkylgruppe

$R^3$ für eine $C_xH_{2x}$- oder $C_xH_{2x-2}$-Gruppe, in welcher x für eine ganze Zahl von 2 bis 4 steht und

$R^4$ für eine $C_yH_{2y}$-Gruppe, in welcher y für eine ganze Zahl von 3 bis 7 steht.

Von den Verbindungen der Formeln I, II und III sind die Verbindungen bevorzugt in denen $R^1$ bis $R^7$ gleich sind und z.B. eine Methyl-, Ethyl-, Propyl-, Isopropyl-, n-Butyl-, Isobutyl-, sek.-Butyl-, tert. -Butyl, Pentyl-, Hexyl- oder Cyclohexylgruppe bedeuten und in denen $R^8$ bis $R^{10}$ gleich sind und z.B. eine Ethylen-, 1,2-Propylen-, 1,3-Propylen-, 1,2-Butylen, 1,3-Butylen, 1,4-Butylen-, 2,3-Butylen-, 1,4-Pentylen oder 2,3-Pentylgruppe bedeuten. .

Besonders bevorzugt sind im Falle der Formel I Verbindungen mit x = 0 bis 5, im Falle der Formel II Verbindungen mit x = 0 bis 5 und y = 0, und im Falle der Formel III Verbindungen, in denen x, y und z eine Zahl von 0 bis 5 bedeuten.

Bei Verbindungen der Formel I sind besonders solche bevorzugt, die aus im technischen Maßstab herstellbaren Alkylendi- und polyaminen (s. Ullmanns Encyclopädie der technischen Chemie, 4. Aufl. 1974, Bd. 7, S. 382 - 383) durch Peralkylierung der Wasserstoffatome am Stickstoff gut zugänglich sind (s. Houben-Weyl, 3. Aufl. 1957, Bd. 11/1, S. 641 - 645). Als Beispiele seien insbesondere genannt: Tetramethylethylendiamin, Pentamethyldiethylentriamin, Hexamethyltriethylentetramin, Heptamethyltetraethylenpentamin, Tetramethylpropylendiamin-1,2, Tetramethylpropylendiamin-1,3, Pentamethyldipropylendiamin, Hexamethyl-tripropylentriamin.

Bei den Verbindungen der Formel II sind besonders bevorzugt: 1,4-Dialkylpiperazine, wie beispielsweise 1,4-Dimethylpiperazin oder 1,4-Diethylpiperazin, und peralkylierte Aza-Kronenether wie Tetramethyl-1,5,9,13-tetraazacyclo-hexadecan oder Hexamethyl-1,4,7,10,13,16-hexaazacyclo-octadecan.

Von den peralkylierten, bicyclischen Di- und Polyaminen der Formel III sei wegen seiner guten technischen Zugänglichkeit insbesondere das Triethylentriamin (1,4-Diazabicyclo [2,2,2]-octan) hervorgehoben.

Von den Verbindungen der Formel IV sind solche bevorzugt in denen $R^1$ eine Methyl-, Ethyl-, Propyl-, Isopropyl-, n-Butyl-, Isobutyl-, sek.-Butyl-, tert.-Butyl-, Pentyl-, Hexyl oder Cyclohexylgruppe bedeutet, $R^2$ Wasserstoff oder eine Methyl-, Ethyl-, Propyl-, Isopropyl-, n-Butyl-, sek.-Butyl-, tert.-Butyl-, Pentyl-, Hexyl- oder Cyclohexylgruppe ist (die gleich oder verschieden von $R^1$ sein kann) und $R^3$ für eine Vinylengruppe oder eine 1,3-Propylengruppe steht.

Als Beispiele für Verbindungen der Formel IV seien wegen ihrer guten technischen Zugänglichkeit besonders hervorgehoben :

1-Methyl-1H-imidazol, 1,2-Dimethyl-1H-imidazol und 1,4,5,6-Tetrahydro-1,2-dimethyl-pyrimidin.

Von den Verbindungen der Formel V sind wegen ihrer leichten Zugänglichkeit solche bicyclischen Amidine bevorzugt, in deren Formel $R^3$ eine Ethylen- oder 1,3-Propylengruppe und $R^4$ eine 1,3-Propylen-, 1,4-Butylen-, 1,5-Amylen- oder 1,7-Heptylengruppe bedeuten. Als Beispiele seien genannt :

2,3,5,6-Tetrahydro-7H-pyrrolo-[1,2-a]-imidazol, ($x = 2$ ; $y = 3$)
2,3,4,6,7,8-Hexahydro-pyrrolo-[1,2-a]-pyrimidin, ($x = 3$ ; $y = 3$)
2,3,4,5,7,8-Hexahydro-9H-pyrrolo-[1,2-a]-[1,3]-diazepin ($x = 4$ ; $y = 3$)
3,4,6,7,8,9-Hexahydro-2H-pyrido-[1,2-a]-pyrimidin ($x = 3$ ; $y = 4$)
2,3,4,6,7,8,9,10-Octahydro-pyrimido-[1,2-a]-azepin ($x = 3$ ; $y = 5$)
2,3,4,6,7,8,9,10,11,12-Decahydro-pyrimido-[1,2-a]-azonin ($x = 3$ ; $y = 7$)

Besonders bevorzugt sind :

2,3,4,6,7,8-Hexahydropyrrolo-[1,2-a]-pyrimidin ($x = 3$ ; $y = 3$) und
2,3,4,6,7,8,9,10-Octahydro-pyrimido-[1,2-a]-azepin ($x = 3$ ; $y = 5$)

Die erfindungsgemäß zu verwendenden Katalysatoren werden in einer Menge von 0,000 1 bis 0,05 Mol, vorzugsweise 0,000 5 bis 0,02 Mol je Mol Phenol eingesetzt.

In dem erfindungsgemäßen Verfahren werden Phenol und Paraformaldehyd im Molverhältnis 5 : 1 bis 15 : 1, vorzugsweise im Molverhältnis 8 : 1 bis 12 : 1 eingesetzt.

Die erfindungsgemäß zu verwendenden Katalysatoren können den Reaktionskomponenten zu Reaktionsbeginn mit einem Male zugesetzt werden. Es ist auch möglich, zu Reaktionsbeginn erst einen Teil des Katalysators zuzusetzen und den Rest kontinuierlich oder intermittierend im Verlauf der Reaktion nachzusetzen.

Das erfindungsgemäße Verfahren wird vorteilhafterweise bei Temperaturen von 25 bis 125 °C, vorzugsweise 40 bis etwa 100 °C durchgeführt. Die Reaktionszeit liegt im allgemeinen zwischen 0,5 und 10 Stunden.

Die Aufarbeitung des Reaktionsgemisches kann, wie in der DE-OS 27 09 075 beschrieben, durch teilweises Abdestillieren des überschüssigen Phenols und Isolierung der 2- und 4-Hydroxy-benzylalkohole durch Gegenstromextraktion erfolgen.

## Beispiele 1 bis 10

Zur Durchführung der Versuche wurden jeweils 10 Mol Phenol und 1 Mol Paraformaldehyd unter Zusatz der in Tabelle angegebenen Menge des in der Tabelle angegebenen Katalysators unter Rühren auf die in der Tabelle angegebene Reaktionstemperatur erhitzt. Die Reaktionsmischung wurde für die in der Tabelle angegebene Reaktionszeit auf der Reaktionstemperatur gehalten.

Die Umsetzung wurde beendet, wenn der polarographisch bestimmte Formaldehyd-Umsatz 85 bis 98 % betrug. Die Zusammensetzung des auf diese Weise erhaltenen Reaktionsgemisches wurde durch Hochdruckflüssigkeit-chromatographie bzw. nach Überführung der Komponenten des Reaktionsgemisches in ihre Trimethylsilylderivate mittels N-Methyl-N-trimethylsilyl-acetamid durch Gaschromatographie bestimmt.

Die Analyse der bei den Umsetzungen angefallenen Hydroxybenzylalkohol-Gemische ergab die in der Tabelle angegebenen Verhältnisse an 2- und 4-Hydroxybenzylalkohol.

(Siehe die Tabelle, Seite 5)

Tabelle

| Beispiel Nr. | Katalysator [$pK_a$-Wert bei 20 °C in $H_2O$] (Mol/Mol Phenol) | Reaktions-temp. (°C) | Reaktions-zeit [h] für (%) $CH_2O$-Umsatz | Hydroxybenzylalkoholgemisch Gehalt (%) an | | |
|---|---|---|---|---|---|---|
| | | | | Ausbeute (% d. Th.) bez. auf einges. $CH_2O$ | 2-Hydro-xybenzyl-alkohol | 4-Hydro-xybenzyl-alkohol |
| 1 | Tetramethylethylendiamin [9,2] (0,002) | 70 | 8 (98) | 82 | 59 | 41 |
| 2 | Pentamethyldiethylentriamin [9,4] (0,002) | 70 | 3 (98) | 85 | 54 | 46 |
| 3 | 1,4-Dimethylpiperazin [7,8] (0,002) | 70 | 8 (85) | 67 | 64 | 36 |
| 4 | Triethylendiamin [8,2] (0,002) | 70 | 9 (98) | 85 | 60 | 40 |
| 5 | 1-Methyl-1H-imidazol [6,8] (0,002) | 70 | 8 (89) | 73 | 56 | 44 |
| 6 | 1,2-Dimethyl-1H-imidazol [7,9] (0,002) | 70 | 7 (97) | 85 | 57 | 43 |
| 7 | 1,4,5,6-Tetrahydro-1,2-dimethylpyrimidin [10,2] (0,002) | 70 | 3 (98) | 85 | 54 | 46 |
| 8 | 2,3,4,6,7,8-Hexahydro-pyrolo [1,2-a]-pyrimidin [9,6] (0,02) | 70 | 4 (98) | 84 | 53 | 47 |
| 9 | 2,3,4,6,7,8-Hexahydro-pyrrolo [1,2-a]-pyrimidin [9,6] (0,0008) | 80 | 3,5 (98) | 83 | 53 | 47 |
| 10 | 2,3,4,6,7,8,9,10-Octahydro-pyrimido [1,2-a]-azepin [9,8] (0,00066) | 90 | 1,3 (98) | 85 | 52 | 48 |
| 11* | Hexamethylentetramin [6,3] (0,002) | 70 | 2 (98) | 70 | 70 | 30 |
| 12* | Kaliumhydroxid (0,002) | 60 | 5 (98) | 85 | 70 | 30 |

* Vergleichsbeispiele

**Ansprüche**

1. Verfahren zur Herstellung eines Gemisches aus 2- und 4-Hydroxybenzylalkohol durch Umsetzung von Phenol mit Paraformaldehyd in Gegenwart basischer Katalysatoren dadurch gekennzeichnet, daß man als Katalysatoren Verbindungen verwendet, die zwei oder mehr tertiäre Stickstoffatome im Molekül und einen $pK_a$-Wert $\geq 6,5$ (gemessen bei 20 °C in Wasser) aufweisen.

2. Verfahren nach Anspruch 1, dadurch gekennzeichnet, daß man als Katalysator peralkylierte offenkettige, monocyclische oder bicyclische Di- oder Polyamine verwendet.

3. Verfahren nach Anspruch 1 und 2, dadurch gekennzeichnet, daß man als Katalysator Tetramethylethylendiamin, Pentamethyldiethylentriamin, Hexamethyltriethylentetramin, 1-4-Dimethylpiperazin oder Triethylentriamin verwendet.

4. Verfahren nach Anspruch 1, dadurch gekennzeichnet, daß man als Katalysator N,N'-substituierte mono- oder bicyclische Amidine verwendet.

5. Verfahren nach Anspruch 1 und 4, dadurch gekennzeichnet, daß man als Katalysator 1-Methyl-1H-imidazol, 1,2-Dimethyl-1H-imidazol, 1,4,5,6-Tetrahydro-1,2-dimethyl-pyrimidin, 2,3,4,6,7,8-Hexahydro-pyrrolo-[1,2-a]-pyrimidin oder 2,3,4,6,7,8,9,10-Octahydro-pyrimido-[1,2-a]-azepin verwendet.

**Claims**

1. Process for the preparation of a mixture of 2- and 4-hydroxybenzyl alcohol by reaction of phenol with paraformaldehyde in the presence of basic catalysts, characterised in that compounds which contain two or more tertiary nitrogen atoms in the molecule and have a $pK_a$ value $\geq 6.5$ (measured at 20 °C in water) are used as the catalysts.

2. Process according to Claim 1, characterised in that peralkylated open-chain monocyclic or bicyclic di- or poly-amines are used as the catalyst.

3. Process according to Claims 1 and 2, characterised in that tetramethylethylenediamine, pentamethyldiethylenetriamine, hexamethyltriethylenetetramine, 1,4-dimethylpiperazine or triethylenetriamine is used as the catalyst.

4. Process according to Claim 1, characterised in that N,N'-substituted monocyclic or bicyclic amidines are used as the catalyst.

5. Process according to Claims 1 and 4, characterised in that 1-methyl-1H-imidazole, 1,2-dimethyl-1H-imidazole, 1,4,5,6-tetrahydro-1,2-dimethyl-pyrimidine, 2,3,4,6,7,8-hexahydro-pyrrolo-[1,2-a]-pyrimidine or 2,3,4,6,7,8,9,10-octahydro-pyrimido-[1,2-a]-azepine is used as the catalyst.

**Revendications**

1. Procédé de fabrication d'un mélange d'alcool 2- et 4-hydrobenzylique par réaction du phénol avec la paraformaldéhyde en présence de catalyseurs basiques, caractérisé en ce qu'on utilise comme catalyseurs des composés qui présentent deux ou plusieurs atomes d'azote tertiaires dans la molécule et une valeur de $pK_a$ égale ou supérieure à 6,5 (mesurée à 20 °C dans de l'eau).

2. Procédé selon la revendication 1, caractérisé en ce qu'on utilise comme catalyseur des di- ou polyamines à chaîne·ouverte, monocycliques ou bicycliques peralcoylées.

3. Procédé selon les revendications 1 et 2, caractérisé en ce qu'on utilise comme catalyseur de la tétraméthyléthylène diamine, pentaméthyldiéthylène triamine, hexaméthyltriéthylène tétramine, 1,4-diméthylpipérazine ou triéthylène triamine.

4. Procédé selon la revendication 1, caractérisé en ce qu'on utilise comme catalyseur des amidines mono- ou bicycliques N,N'-substituées.

5. Procédé selon les revendications 1 et 4, caractérisé en ce qu'on utilise comme catalyseur du 1-méthyl-1H-imidazol, 1,2-diméthyl-1H-imidazol, 1,4,5,6-tétrahydro-1,2-diméthyl-pyrimidine, 2,3,4,6,7,8-hexahydro-pyrrolo-[1,2-a]-pyrimidine ou 2,3,4,6,7,8,9,10-octahydro-pyrimido-[1,2-a]-azépine.